Europäisches Patentamt

European Patent Office

Office européen des brevets

(19)

(11) Publication number: 0 327 934
A1

## EUROPEAN PATENT APPLICATION

(12)

(21) Application number: 89101679.2

(22) Date of filing: 01.02.89

(51) Int. Cl.⁴: **G01N 33/52** , **G01N 33/70** , **G01N 33/72** , **G01N 33/92** , **G01N 21/29**

(30) Priority: 08.02.88 IT 1933488

(43) Date of publication of application:
16.08.89 Bulletin 89/33

(84) Designated Contracting States:
AT BE CH DE ES FR GB GR IT LI LU NL SE

(71) Applicant: **Beli, Raffaele**
**Via Lupia, 5**
**Lecce(IT)**

Applicant: **Corchia, Arturo**
**Via delle Lenze, 3**
**Pisa(IT)**

(72) Inventor: **Beli, Raffaele**
**Via Lupia, 5**
**Lecce(IT)**
Inventor: **Corchia, Arturo**
**Via delle Lenze, 3**
**Pisa(IT)**

(74) Representative: **Gervasi, Gemma**
**NOTARBARTOLO & GERVASI Srl Viale**
**Bianca Maria 33**
**I-20122 Milan(IT)**

(54) **Device and method for carrying out extemporaneous quantitative diagnostic tests on the whole blood.**

(57) A device for carrying out extemporaneous quantitative diagnostic tests on the whole blood which comprises:

a) a rigid plastics strip, to the end of which there is cemented a substrate containing a reactive substance which undergoes a selective colour reaction respectively with the substance to be determined in the blood, and

b) a visual colorimetric reference scale specific for each of the examined substances and possibly corrected so as to directly read the content of the examined substance in the plasma from the test carried out the whole blood;

and the method thereof.

# DEVICE AND METHOD FOR CARRYING OUT EXTEMPORANEOUS QUANTITATIVE DIAGNOSTIC TESTS ON THE WHOLE BLOOD

## FIELD OF THE INVENTION

This invention relates to a device and the relative method for carrying out extemporaneous quantitative diagnostic tests on the whole blood.

More particularly, the invention relates to a device and a method based on colour reactions for the extemporaneous determination of uricemia, azotemia, creatininemia and cholesterolemia in the whole blood.

The device and method of the present invention are of considerable use in checking patients who, after initial scrupulous control of their illness, tend to lose this control as they are unwilling to periodically attend the analysis laboratory because of the burocracy involved and the risk of losing their entire working day.

In this respect the tests according to the invention can be carried out in the home by a simple manual procedure and require only a short time.

## PRIOR ART

Elements in the form of tapes are known comprising a transparent support and a layer containing a reactive substance which changes colour by reaction with one of the components of the liquid under examination, such as the blood, the quantitative determination being done spectrophotometrically (G.B. patent 1,440,464). Strips are also known for diagnostic tests for determining blood and hemoglobin in biological materials, these comprising an unsized paper support impregnated with an organic hydroperoxide, a buffer acid, a chromogen, a wetting agent, a filmogenic polymer substance and an agent able to accelerate the activity of the peroxide.

The method is suitable for example for determining blood in the urine (G.B. patent 1,490,380).

The various known methods do not however satisfy the require ment for the extemporaneous determination in the home of uricemia, azotemia, creatininemia and cholesterolemia.

The great importance of tests relating to these hematic indices is illustrated by the following considerations.

The uric acid content in the blood is an index of purine metabolism and of renal functionality; the creatininemia is the most reliable hematochemical index of renal insufficiency; the azotemia is an index of renal functionality but can be influenced by alterations in the circulation of both general and local kind (hypotension, shock, dehydration, vomit and diarrhea), by diet, by illnesses accompanied by relevant tissue destruction and by the use of certain drugs (cortisones, tetracyclines); the cholesterolemia increases in essential hypercholesterolemia, in mixed hyperlipemias, in secondary hyperlipemias (nephrotic syndrome, myxedemia, diabetes melitus, biliary cirrhosis, obstructive jaundice) and in Cushing's disease.

Determination of blood cholesterol is very important as a "humoral indication" of possible atherogenic tendency.

## SUMMARY OF THE INVENTION

The aim of the present invention is to provide a device comprising:

a) a rigid plastics strip, to the end of which there is cemented a substrate containing a reactive substance which undergoes a selective colour reaction respectively with the substance to be determined in the blood; and

b) a visual colorimetric reference scale specific for each of the examined substances and possibly corrected so as to directly read the content of the examined substance in the plasma from the test carried out on the whole blood.

A further object of the present invention is to provide a method for carrying out the test comprising the following stages:

1) a drop of blood is withdrawn from the finger tip,

2) the whole blood is poured onto the substrate supported by the plastics strip and containing the reactive substance, and after one minute is removed by dry cotton wool,

3) a visual reading is made by comparison with said colorimetric scale one minute after removing the blood.

## DETAILED DESCRIPTION OF THE INVENTION

A substrate able to undergo a colour reaction with the substance sought in the blood is cemented to the end of a plastics strip.

The reactive substances used for incorporation into the substrate are: phosphotungstic acid for determining the uricemia, 1:1 w/w picric acid with sodium bicarbonate for determining the creatininemia, diacetyl monoxime for determining the azotemia, and the Liebermann-Burchard reagent for determining the cholesterolemia.

The substrate containing the reactive substance is prepared by depositing on unsized paper a layer of the reactive substance mixed with an inert substance able to give an omogeneous mixture of a pasty consistency, such as glycerol, PVC, gelatine and carboxymethylcellulose. Preferably a stabilizing substance, such as magnesium trisilicate, is also added.

In a preferred embodiment of the invention one part by weight of the reactive substance is mixed with one part by weight of magnesium trisilicate in form of a saturated solution and with two parts by weight of glycerol. The paste obtained is applied to the unsized paper to a thickness of 1-2 mm and is kept in a dry dark environment for 4-8 hours.

The substrate prepared in this manner is then cut preferably into small rectangles of 20-50 mm$^2$ each and each of these rectangles is glued by means of silicone or another inert glue onto a rigid plastics strip.

We have surprisingly found that when the substrate prepared as described comes into contact with the whole blood, it allows the substance under examination to react with the reactive substance while at the same time preventing interference with the chromogenic agents of the blood.

The procedure used in preparing said substrate can be carried out either manually or by industrial processes.

Separately, colorimetric scales are constructed on the basis of the colour reactions of the substances incorporated in said substrates, for progressively increasing concentrations of the examined substances in the whole blood. It is advisable to repeat the colorimetric scale calibration for each stock of substrates, whether to be worked manually or industrially.

The colorimetric scale calibration is done by two methods, each having a different objective, namely either by introducing suitable correction factors by means of which the content of the examined substance in the plasma is read off directly even though the test is made on the whole blood, or without any correction in which case the result represents the total concentration of the examined substance in the whole blood.

The correction factor derives from the difference between the content of the examined substance in the whole blood and its content in the plasma.

For example, considering that the uric acid in the plasma represents 72.8% of that contained in the whole blood, the value obtained by the test when carried out on the whole blood has to be reduced by 27.2%.

For the creatininemia the value obtained for the whole blood has to be reduced by 1.2%, whereas for the azotemia the reduction is 57.0%.

Obviously, if the figure of interest is that relative to the whole blood, such as when determining total cholesterolemia, no correction is made to the colorimetric scale.

The method for carrying out the tests according to the invention comprises the following stages:

1) a drop of blood is withdrawn from the finger tip,

2) the whole blood is poured onto the substrate supported by the plastics strip and containing the reactive substance, and after one minute is removed by dry cotton wool,

3) a visual reading is made by comparison with said colorimetric scale one minute after removing the blood.

The reaction of the blood on said substrate containing the reactive substance is conducted at a temperature of between 18 and 30° C.

The tests for uricemia, creatininemia, azotemia and cholesterolemia conducted in accordance with the invention have all been checked by comparison with determinations made in the laboratory by the autoanalyzer system on 50 blood samples from patients having both normal and altered indices, the results being as follows.

In the uricemia determination test, 90% of the cases gave values which were perfectly superimposable on those determined by the autoanalyzer system whereas in the remaining 10% of cases the values showed a difference of not more than 20%.

In the creatininemia determination test, 93% of the cases gave values which were perfectly superimposable on those determined by the autoanalyzer system whereas in the remaining 7% of cases the values showed a difference of not more than 20%.

In the azotemia determination test the values obtained were all practically superimposable on those obtained by the autoanalyzer system.

In the total cholesterolemia determination test the results were completely superimposable on those obtained by autoanalyzer system.

## Claims

1. A device for carrying out extemporaneous quantitative diagnostic tests on the whole blood comprising:

a) a rigid plastics strip, to the end of which there is cemented a substrate containing a reactive substance which undergoes a selective colour reaction respectively with the substance to be determined in the blood; and

b) a visual colorimetric reference scale specific for each of the examined substances and possibly corrected so as to directly read the content of the examined substance in the plasma from test carried out on the whole blood.

2. A device according to claim 1, characterised by the fact that said substrate contains a reactive substance selected from the group consisting of phosphotungstic acid for determining the uricemia, 1:1 w/w picric acid with sodium bicarbonate for determining the creatininemia, diacetyl monoxime for determining the azotemia, and the Liebermann-Buchard reagent for determining the cholesterolemia.

3. A device as claimed in claim 2, characterised in that said substrate containing phosphotungstic acid is prepared by depositing on unsized paper a 1-2 mm thick layer of phosphotungstic acid mixed with an inert substance able to give an omogeneous mixture of a pasty consistency and with a stabilizing substance.

4. A device as claimed in claim 2, characterised in that said colorimetric scale for uricemia is constructed on the basis of the colour reaction between phosphotungstic acid and progressively increasing concentrations of uric acid in the whole blood.

5. A device as claimed in claim 2, characterised in that said correction to the colorimetric scale for uricemia is obtained by reducing by 27.2% the value obtained in the test when carried out on the whole blood.

6. A device as claimed in claim 2, characterised in that said substrate containing picric acid and sodium bicarbonate is prepared by depositing on unsized paper a 1-2 mm thick layer of these substances mixed with an inert substance able to give an omogeneous mixture of a pasty consistency and with a stabilizing substance.

7. A device as claimed in claim 2, characterised in the said colorimetric scale for creatininemia is constructed on the basis of the colour reaction between picric acid in the presence of sodium bicarbonate, and progressively increasing concentrations of creatinine in the whole blood.

8. A device as claimed in claim 2, characterised in that said correction to the colorimetric scale for creatininemia is obtained by reducing by 1.2% the value obtained in the test when carried out on the whole blood.

9. A device as claimed in claim 2, characterised in that said substrate containing diacetylmonoxime is prepared by depositing on unsized paper a 1-2 mm thick layer of diacetylmonoxine mixed with an inert substance able to give an omogeneuos mixture of a pasty consistency and with a stabilizing substance.

10. A device as claimed in claim 2, characterised in that said colorimetric scale for azotemia is constructed on the basis of the colour reaction between diacetylmonoxime and progressively increasing concentrations of urea in the whole blood.

11. A device as claimed in claim 2, characterised in that said correction to the colorimetric scale for azotemia is obtained by reducing by 57.0% the value obtained in the test when carried out on the whole blood.

12. A device as claimed in claim 2, characterised in that said substrate containing Liebermann-Buchard reagent is prepared by depositing on unsized paper a 1-2 mm thick layer of said reagent mixed with an inert substance able to give an omogeneous mixture of a pasty consistency and with a stabilizing substance.

13. A device as claimed in claims 3, 6, 9, and 12, characterized in that said inert substance is glycerol, PVC, gelatine and carboxymethylcellulose, and that stabilizing substance is magnesium trisilicate.

14. A device as claimed in claim 2, characterised in that said colorimetric scale for cholesterolemia is constructed on the basis of the colour reaction between Liebermann-Buchard reagent and progressively increasing concentrations of cholesterol in the whole blood.

15. A device as claimed in claim 2, characterised in that said substrate containing said reactive substance is cut into rectangles of 20-50 mm$^2$ area and is cemented by means of silicone or other inert adhesive to said rigid plastics strip.

16. A method for carrying out extemporaneous quantitative diagnostic tests on whole blood by the device comprising:

a) a rigid plastics strip, to the end of which there is cemented a substrate containing a reactive substance which undergoes a selective colour reaction respectively with the substance to be determined in the blood, and

b) a visual colorimetric reference scale specific for each of the examined substances and possibly corrected so as to directly read the content of the examined substance in the plasma from test carried out on the whole blood, characterised by: 1) withdrawing a drop of blood from the finger tip,

2) pouring the whole blood onto the substrate supported by the plastics strip and containing the reactive substance, and after one minute removing it by dry cotton wool,

3) making a visual reading by comparison with said colorimetric scale.

17. A method as claimed in claim 16, characterised in that the reaction of the whole blood on said substrate containing the reactive substance is carried out at a temperature of between 18 and 30°C.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-3 891 507 (C.B. BREUER) <br> * Figures 1,2; columns 2-4; column 5, lines 35-45; claims 1,2 * | 1 | G 01 N 33/52 <br> G 01 N 33/70 <br> G 01 N 33/72 <br> G 01 N 33/92 <br> G 01 N 21/29 |
| Y | US-A-4 125 372 (SHOJI KAWAI) <br> * Figures 1,2; column 2, lines 11-43; column 5, lines 59-68; column 6, lines 1-9; claims 1-6 * | 1 | |
| Y | RESEARCH DISCLOSURE, no. 163, November 1977, pages 23-28, disclosure no. 16329, Havant, Hants, GB; "Analytical element for clinical analysis" <br> * Page 27, column 1, example 1; page 28, column 1, example 4 * | 1 | |
| Y | US-A-3 723 063 (G.M. JONES et al.) <br> * Columns 3,4; example 1; columns 5,6; examples 5,6 * | 1 | |
| A | EP-A-0 131 194 (MILES LABORATORIES) <br> * Figure 1; claims * | 1 | **TECHNICAL FIELDS SEARCHED (Int. Cl.4)** |
| A | CH-A- 416 156 (C.F. BOEHRINGER) <br> * Page 1, column 2, lines 52-55; page 2, column 2, lines 1-106 * | 1 | G 01 N |
| A | EP-A-0 198 628 (SMITHKLINE DIAGNOSTICS) <br> * Figures 5,6,8; page 4, lines 9-20 * | 1 | |
| A | EP-A-0 244 094 (UNILEVER PLC) <br> * Figure 1; claims * | 1 | |

The present search report has been drawn up for all claims

| Place of search | Date of completion of the search | Examiner |
|---|---|---|
| THE HAGUE | 19-05-1989 | MEYLAERTS H. |